# EUROPEAN PATENT SPECIFICATION

(11) **EP 1 738 696 B1**
(45) Date of publication and mention of the grant of the patent: **20.08.2008**
(21) Application number: 06253249.4
(22) Date of filing: 22.06.2006
(51) Int. Cl.: A61B 17/12

(54) **Stretch resistant embolic coil delivery system with mechanical release mechanism**
Einführvorrichtung für eine ausdehnungswiderstandsfähige Emboliespiralfeder mit einem mechanischen Auslösemechanismus
Dispositif de déploiement d'une spirale embolique résistante à l'étirement comportant un mécanisme de libération mécanique

(30) Priority: 30.06.2005 US 172198
(43) Date of publication of application: 03.01.2007
(73) Proprietor: Cordis Neurovascular, Inc., Miami Lakes, Florida 33014 (US)
(72) Inventor: Mitelberg, Vladimir, Aventura, FL 33180 (US); Jones, Donald K., Lauderhill, FL 33351 (US); Lorenzo, Juan A., Davie, FL 33328 (US)
(74) Representative: Belcher, Simon James

(56) References cited:
- EP-A1- 0 754 435
- EP-A1- 0 832 607
- US-A- 5 263 964
- US-A- 5 925 059
- US-A1- 2002 165 569
- US-A1- 2004 034 363

## Description

The present invention relates to a medical device for placing a stretch resistant embolic device at a predetermined site within a vessel of the human body, and more particularly, relates to a catheter-based deployment system for delivering an embolic device. This device is particularly suited to transport an embolic device, such as a stretch resistant embolic coil, through the tortuous vasculature of the human brain to a selected site within the vessel or within an aneurysm.

For many years, flexible catheters have been used to place various devices within the vessels of the human body. Such devices include dilation balloons, radiopaque fluids, liquid medications, and various types of occlusion devices such as balloons and embolic coils. Examples of such catheter-based devices are disclosed in US-5108407, entitled, "Method and Apparatus for Placement of an Embolic Coil" and US-5122136, entitled, "Endovascular Electrolytically Detachable Guidewire Tip For The Electroformation Of Thrombus In Arteries, Veins, Aneurysms, Vascular Malformations And Arteriovenous Fistulas." These patents disclose catheter-based devices for delivering embolic coils to preselected positions within vessels of the human body in order to treat aneurysms, or alternatively, to occlude blood vessels at a particular location.

Coils which are placed in vessels may take the form of helically wound coils, or alternatively, may take the form of randomly wound coils, coils wound within coils or other such coil configurations. Examples of various coil configurations are disclosed in US-5334210, entitled, "Vascular Occlusion Assembly" and US-5382259 entitled, "Vasoocclusion Coil with Attached Tubular Woven or Braided Fibrous Covering." Embolic coils are generally formed of a radiopaque metallic material, such as platinum, gold, tungsten, or alloys of these metals. Often, several coils are placed at a given location to occlude the flow of blood through the vessel, or aneurysm, by promoting thrombus formation at the particular site.

In the past, embolic coils have been placed within the distal end of a catheter. When the distal end of the catheter is properly positioned, the coil may then be pushed out of the end of the catheter with a pusher member to release the coil at the desired location. This procedure for placement of an embolic coil is conducted under fluoroscopic visualization such that the movement of the coil through the vasculature of the body may be monitored and the coil placed at the desired location.

Another procedure involves the use of glue or solder for attaching the coil to a guidewire, which in turn, is placed within a flexible catheter for positioning the coil within the vessel at a preselected position. Once the coil is in the desired position, the coil is held in position by the catheter and the guidewire is pulled proximally to thereby cause the coil to become detached from the guidewire and released from the catheter. Such a coil positioning system is disclosed in US-5263964 entitled, "Coaxial Traction Detachment Apparatus and Method."

Still another coil positioning procedure is that of having a catheter with a socket at the distal end of the catheter for retaining a ball which is, in turn, bonded to the proximal end of the coil. The ball, which is generally larger in diameter than the outside diameter of the coil, is placed in the socket within the lumen at the distal end of the catheter and the catheter is then moved into a vessel in order to place the coil at a desired position. Once the position is reached, a pusher wire with a piston at the end thereof is pushed distally from the proximal end of the catheter to push the ball out of the socket in order to release the coil at the desired position. Such a system is disclosed in US-5350397, entitled, "Axially Detachable Embolic Coil Assembly."

Another procedure for placing an embolic coil within a vessel is that of using a heat releasable adhesive bond for retaining the coil at the distal end of the catheter. One such system uses laser energy transmitted through a fiber optic cable to apply heat to the adhesive bond in order to release the coil from the end of the catheter. Such a procedure is disclosed in US-5108407, entitled "Method and Apparatus for Placement of an Embolic Coil."

Yet another coil deployment system incorporates a catheter having a lumen throughout the length of the catheter and a distal tip for retaining the coil for positioning the coil at a preselected site. The distal tip of the catheter is formed of a material which exhibits the characteristic that when the lumen of the catheter is pressurized the distal tip expands radially to release the coil at the preselected site. Such a deployment system is disclosed in US-6113622, entitled, "Embolic Coil Hydraulic Deployment System."

Still another coil deployment system incorporates an interlocking mechanism on the coil. The interlocking end on the embolic coil couples with a similar interlocking mechanism on a pusher assembly. A control wire which extends through the locking mechanism secures the coil to the pusher assembly. The pusher assembly and embolic coil are initially disposed within the lumen of a catheter. When the embolic coil is pushed out of the end of the catheter for placement, the control wire is retracted and the coil disengages from the pusher assembly. Such a deployment system is disclosed in US-5925059, entitled, "Detachable Embolic Coil Assembly."

US-A-2002/0165569, entitled, "Intravascular Device Deployment Mechanism Incorporating Mechanical Detachment." discloses a coil deployment system according to the preamble of claim 1. This deployment system incorporates an embolic device detachably mounted on the distal portion of a pusher member and held in place with a connector thread or fiber. The fiber passes through a cutter member that may be activated to cut the connector fiber. Once the connector fiber is cut, the embolic device is released.

Still another coil deployment system incorporates an embolic device with a stretch resistant member therethrough. The distal end of the stretch resistant member attaches to the embolic coil and the proximal end of the stretch resistant member is detachably mounted on the pusher member through various means such as adhesive, or by a connector fiber adhered to or tied to the pusher member, and is detachable by the application of heat. Such a deployment system is disclosed in US-A-2004/0034363, entitled, "Stretch Resistant Therapeutic Device."

Still another coil deployment system incorporates a pusher wire with a stiff wavy-shaped end segment which is coupled to the embolic coil and is placed in the lumen of the catheter. The coil is advanced through the catheter until it reaches a predetermined site in the vessel at which time the pusher wire is retracted and the embolic coil is released. Such a system is disclosed in US-6203547, entitled, "Vaso-occlusion Apparatus Having A Manipulable Mechanical Detachment Joint And A Method For Using The Apparatus."

A still further embolic device deployment system for placement of an embolic device, or coil, includes a delivery catheter and a flexible pusher member. The embolic device is retained by an interlocking mechanism which includes a detachment member which extends through an aperture in an engagement member which is attached to a pusher member. The engagement member engages a ring on the embolic device. When the detachment member is withdrawn from the aperture of the engagement member, the embolic device is released. One such deployment system is disclosed in European Patent Application No. 06252708.0, entitled, "Stretch Resistant Embolic Coil Delivery System With Mechanical Release Mechanism," filed on 24 May 2006 in the name of the same applicant as the present application.

The present invention is directed toward a vascular occlusive embolic device deployment system for use in placing a stretch-resistant embolic device at a predetermined site within a vessel which includes an elongated flexible catheter, an elongated pusher member having a lumen extending therethrough and being slidably disposed within the lumen of the catheter. The embolic device takes the form of an embolic coil defining a central lumen extending between the proximal and distal ends of the coil. A stretch resistant member, such as a platinum wire, having first and second ends in which the first end of the stretch resistant member is attached to the distal section of the coil and the second end of the stretch resistant member is attached to a retaining ring. An engagement member, preferably having an L-shaped configuration at its distal end, is slidably disposed within the lumen of the pusher member and includes an aperture extending through the distal end thereof. The engagement member extends through the retaining ring of the stretch-resistant embolic device. In addition, the deployment system includes an elongated detachment member which extends from the proximal end of the pusher member, through the lumen of the pusher member and through the aperture of the engagement member such that when the detachment member is pulled proximally the distal end of the detachment member is withdrawn from the aperture of the engagement member to thereby release the embolic device, characterised in that said aperture extends through the distal end of the engagement member such that when said engagement member extends through said retaining ring and when the detachment member extends through said aperture said retaining ring is interlocked onto said engagement member until the detachment member is withdrawn from said aperture releasing said aperture from said retaining ring.

In accordance with another aspect of the present invention, there is provided a deployment system for use in placing an embolic device at a predetermined site within a vessel which includes an elongated flexible catheter, an elongated pusher member being slidably disposed within the lumen of the catheter. The embolic device takes the form of an embolic coil defining a central lumen extending between the proximal and distal ends of the coil. A stretch resistant member having first and second ends in which the first end of the stretch resistant member is attached to the distal section of the coil and the second end of the stretch resistant member is attached to a retaining ring. An elongated engagement member, preferably having an L-shaped configuration at its distal end, is slidably disposed within the pusher member and includes an aperture extending through the distal end thereof. The engagement member extends through the retaining ring of the stretch-resistant embolic device. In addition, the deployment system includes an elongated detachment member which extends from the proximal end of the catheter through the lumen of the catheter and through the aperture of the engagement member such that when the detachment member is pulled proximally the distal end of the detachment member is withdrawn from the aperture of the engagement member to thereby release the embolic device.

In accordance with another aspect of the present invention, the second end of the stretch-resistant member is attached to the proximal section of the coil, as opposed to the retaining ring, to prevent the coil from stretching, and the proximal end of the coil is attached to the retaining ring.

In accordance with another aspect of the present invention, the elongated engagement member has an L-shaped configuration at its distal end. The aperture extends through the distal end of the engagement member which extends through the retaining ring such that when the detachment member extends through the aperture the retaining ring of the embolic device is interlocked onto the engagement member until the detachment member is withdrawn from the aperture.

In accordance with another aspect of the present invention, the aperture has a central axis which extends substantially at a right angle to the central axis of the retaining ring. In addition, the embolic device takes the form of a helically wound embolic coil having a central axis which extends at a right angle to the central axis of the retaining ring. The stretch resistant member is attached to and extends from a distal section to a proximal section of the helically wound coil.

In accordance with still anther aspect of the present invention, the embolic device takes the form of a helically wound coil formed of a plurality of turns of which one turn has a central axis which extends at a right angle to the central axis of the other turns to thereby form the retaining ring.

In addition, the vascular embolic device deployment system preferably includes a retaining clamp mounted on the proximal end of the pusher member, and the detachment member and the engagement member extend from a position proximal of the retaining clamp and through a lumen in the clamp in order that the detachment member and the engagement member may be clamped in a fixed position prior to the release of the embolic device. Upon release of the clamp, the detachment member may be withdrawn from the aperture of the engagement member to thereby release the embolic device.

According to another aspect of the present invention, there is provided a vasooclusive embolic device deployment system for use in placing an embolic device at a predetermined site within a vessel comprising: an elongated flexible deployment catheter having a lumen extending therethrough and having proximal and distal ends; an elongated pusher member having a lumen extending therethrough and having proximal and distal ends and being slidably disposed within the lumen of the deployment catheter; an embolic device which takes the form of an embolic coil being coupled to a retaining ring; an elongated engagement member being slidably disposed within the lumen of the pusher member and having an aperture extending through the distal end thereof, said engagement member extending through said retaining ring; and, an elongated detachment member extending from a position proximal of the proximal end of the pusher member, through the lumen of the pusher member and through the aperture of the engagement member such that when the detachment member is pulled proximally the distal end of the detachment member is withdrawn from the aperture of the engagement member to thereby release the embolic device.

According to another aspect of the present invention, there is provided a vasooclusive embolic device deployment system for use in placing an embolic device at a predetermined site within a vessel comprising: an elongated flexible deployment catheter having a lumen extending therethrough and having proximal and distal ends; an elongated pusher member having proximal and distal ends and being slidably disposed within the lumen of the deployment catheter; an embolic device which takes the form of an embolic coil being coupled to a retaining ring; an elongated engagement member being slidably disposed within the lumen of the deployment catheter and having an aperture extending through the distal end thereof; said engagement member extending through said retaining ring of the embolic device; and, an elongated detachment member extending from a position proximal of the deployment catheter, through the lumen of the catheter and through the aperture of the engagement member such that when the detachment member is pulled proximally the distal end of the detachment member is withdrawn from the aperture of the engagement member to thereby release the embolic device.

According to another aspect of the present invention, there is provided a vasooclusive embolic device deployment system for use in placing an embolic device at a predetermined site within a vessel comprising: an elongated flexible deployment catheter having a lumen extending therethrough and having proximal and distal ends; an elongated pusher member having a lumen extending therethrough and having proximal and distal ends and being slidably disposed within the lumen of the deployment catheter; an embolic device which takes the form of an embolic coil being coupled to a retaining ring; an elongated engagement member slidably disposed within the lumen of the deployment catheter and having an aperture extending through the distal end thereof, said engagement member extending through said retaining ring; and, an elongated detachment member extending from a position proximal of the proximal end of the pusher member, through the lumen of the pusher member and through the aperture of the engagement member such that when the detachment member is pulled proximally the distal end of the detachment member is withdrawn from the aperture of the engagement member to thereby release the embolic device.

According to another aspect of the present invention, there is provided a vasooclusive embolic device deployment system for use in placing an embolic device at a predetermined site within a vessel comprising: an elongated flexible deployment catheter having a lumen extending therethrough and having proximal and distal ends; an elongated pusher member having proximal and distal ends and being slidably disposed within the lumen of the deployment catheter; an embolic device which takes the form of an embolic coil being coupled to a retaining ring; an elongated engagement member slidably disposed within the lumen of the deployment catheter and having an aperture extending through the distal end thereof; said engagement member extending through said retaining ring of the embolic device; and, an elongated detachment member extending from a position proximal of the deployment catheter, through the lumen of the catheter and through the aperture of the engagement member such that when the detachment member is pulled proximally the distal end of the detachment member is withdrawn from the aperture of the engagement member to thereby release the embolic device.

Embodiments of the invention will now be described by way of example with reference to the accompanying drawings, in which:
Figure 1 is an enlarged, partially sectional view of an embodiment of an embolic device deployment system in accordance with the present invention;
Figure 1A is an enlarged, partially sectional view of a second embodiment of an embolic device deployment system in accordance with the present invention;
Figures 2A, 2B and 2C are enlarged, sectional views, illustrating in more detail the coil deployment system of Figure 1;
Figures 3, 3A, 3B, and 3C are enlarged, sectional views of the coil deployment system shown in Figures 1 and 2 illustrating the sequential steps in the advancement of the embolic device, removal of a detachment member, and release of the embolic device.

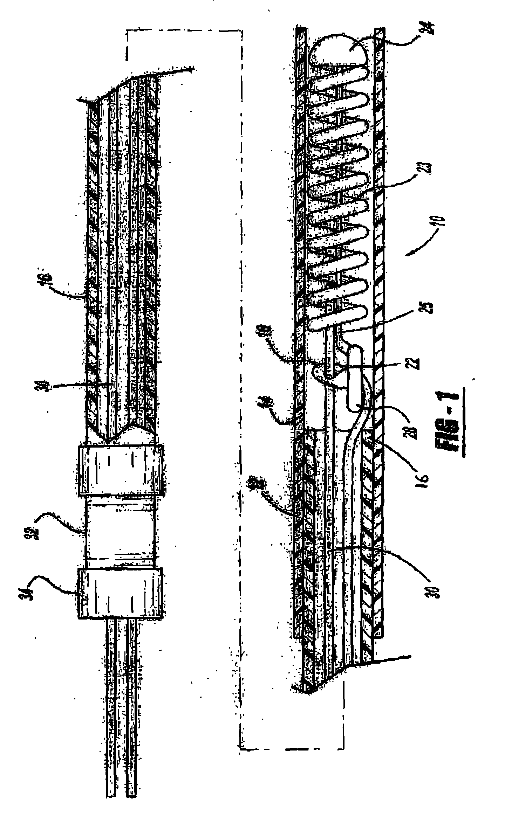

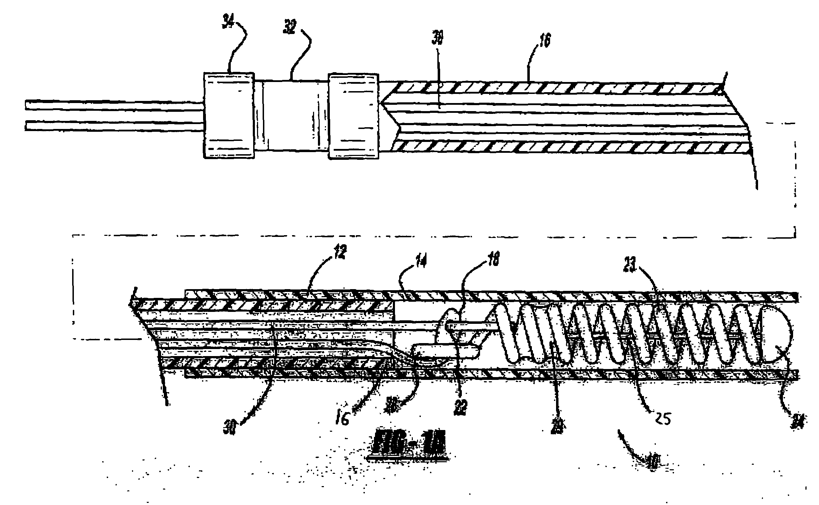

Figure 1 generally illustrates one embodiment of a vascular occlusive embolic device deployment system 10 which includes a sheath introducer 12 having a lumen 14 extending therethrough and having an elongated pusher member 16 slidably disposed within the lumen 14 of the sheath introducer 12. An elongated engagement member 18 is slidably disposed within a lumen of the pusher member 16 and has an aperture 22 extending through the distal end thereof. The central axis of the aperture 22 extends generally parallel to the axis of the pusher member 16. The engagement member 18 is preferably formed of a small diameter resilient wire, such as Nitinol, and includes a flattened distal end having a passageway extending therethrough to form the aperture 22.

The deployment system 10 also includes an embolic device 23, which as illustrated, preferably takes the form of a helically wound embolic coil, which is disposed in the distal section of the sheath introducer 12. While the embolic device as illustrated is shown as a helically wound coil various other types of embolic devices, such as filaments, braids, foams, expandable meshes and stents, could be delivered using the present deployment system and various other coil configurations could be delivered using this system. A weld, or solder, bead 24 is formed at the distal end of the embolic device 23 to provide an atraumatic tip for the embolic device. In addition, the distal end of a stretch-resistant member 25, which preferably takes the form of a platinum wire, is attached to the distal bead 24 and extends proximally through the central lumen of the coil. While the stretch-resistant member preferably takes the form of a platinum wire, other materials or composites such as polymers, metals and ceramics, having a low elongation relative to the coil elongation may also be suitable. Alternatively, the distal end of the stretch-resistant member could be attached to the coil at a more proximal location in the distal section of the coil. The proximal end of the stretch resistant member is then attached to the edge of the retaining ring 28. Preferably, the retaining ring 28 has a central axis which extends at right angles to the central axis of the sheath introducer 12 and also extends at right angles to the central axis of the helically wound embolic coil.

Figure 1A illustrates another variation of the stretch-resistant embolic device 23 in which the distal end of a stretch-resistant member 25 is attached to the bead 24 at the distal end of the coil and the proximal end of the stretch-resistant member 25 is attached to the turns in the proximal section of the coil by use of a weld, or solder, bead 29. With this embodiment, the most proximal end of the coil is attached to the retaining ring 28.

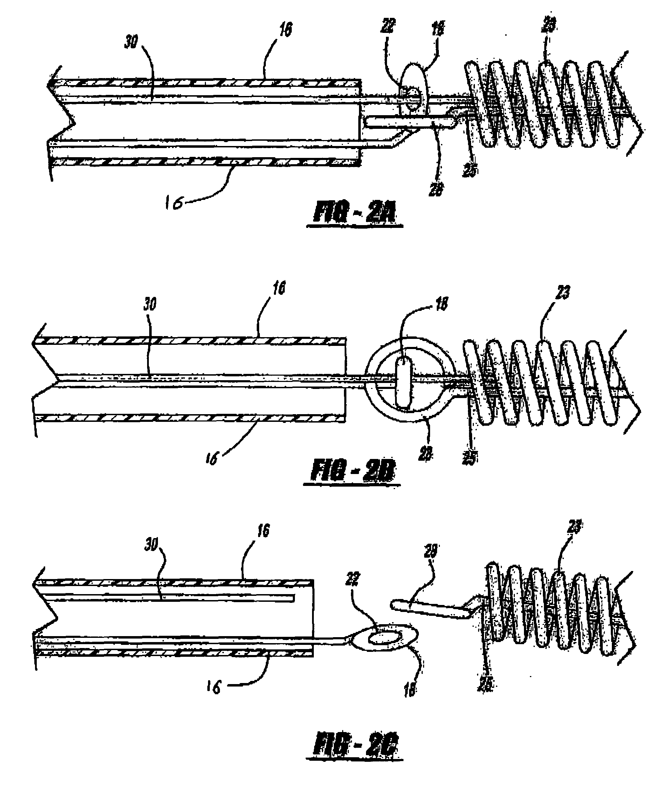

As illustrated in Figures 1, 1A, 2A and 2B, the engagement member 18 extends in a direction parallel to the central axis of the pusher member 16 and extends through the retaining ring 28 and is constrained in a generally L-shaped configuration by a detachment member 30. The elongated detachment member 30 extends from the proximal end of the deployment system 10 and through a lumen in the pusher member and then through the aperture 22 of the engagement member 18 and serves the function of interlocking the embolic device 23 to the pusher member 16 until such time as the detachment member 30 is withdrawn proximally. Alternatively, the configuration of the pusher member 16 may include two or more lumens through which the engagement member 18 and the detachment member 30 are disposed within separate lumens. When the detachment member 30 is withdrawn from the aperture 22, the engagement member 18 returns to its normal straight configuration thereby releasing the retaining ring 28. The detachment member 30 preferably takes the form of a small diameter elongate filament, however, other forms such as wires or tubular structures are also suitable. While the detachment member 30 is preferably formed of nitinol, other metals and materials such as, stainless steel, PTFE, nylon, ceramic or glass fiber and composites may also be suitable.

A Tuohy-Borst type of clamp 32 is mounted on the proximal end of the pusher member 16 and when tightened onto the detachment member 30 and onto the engagement member 18 and serves to prevent movement of the detachment member and the engagement member 18 until such time as the clamping cap 34 is loosened to release the grip onto these members. Figure 2A and 2B illustrate the interlocking arrangement between the embolic device 23 and the pusher member 16 as shown in Figure 1, however, these latter figures illustrate the operation of the deployment system once the pusher member 16 has been moved distally to a position so that the distal end of the pusher member 16 extends slightly out of the distal end of the sheath introducer 12, or a delivery catheter, thereby exposing the embolic device 23. As illustrated in Figure 2C, once the embolic device 23 has been moved out of the end of the sheath introducer 12 the detachment member 30 may be pulled proximally to withdraw the detachment member from the aperture 22 of the engagement member 18 to thereby cause the engagement member to disengage from the retaining ring 28 of the embolic device thereby releasing the embolic device 23 at a preselected position. In operation, once the embolic device 23 is properly positioned, cap 34 of the Touhy-Borst clamp 32 is loosened and the detachment member 30 is withdrawn proximally while holding the engagement member in a fixed position relative to clamp 32 thereby unsecuring the embolic device 23 for release. To enhance the release of the embolic device, the engagement member 18 may be subsequently withdrawn proximally. Alternatively, if desired, the detachment sequence described above and illustrated in Figures 2A, 2B and 2C may be executed while the embolic device 23 is still within the lumen of sheath introducer 12 or a delivery catheter.

One of the important advantages of the present invention is that the embolic device may be placed at a desired location within a vessel, or within an aneurysm, with the configuration of the device deployment system as shown in Figures 2A and 2B. If it is determined that the embolic device is improperly positioned, the embolic device 23 may then be withdrawn from that location and placed at another location, or even removed from the body by first withdrawing the pusher member 16 and the embolic device totally back into the delivery catheter. Once the embolic device has been entirely withdrawn back into the delivery catheter, the catheter may then be moved to a more desirable location and the embolic device may then be released at the new location. With the addition of the stretch resistant member 25, the embolic device may be withdrawn without concern that the coil will stretch and become very difficult to remove.

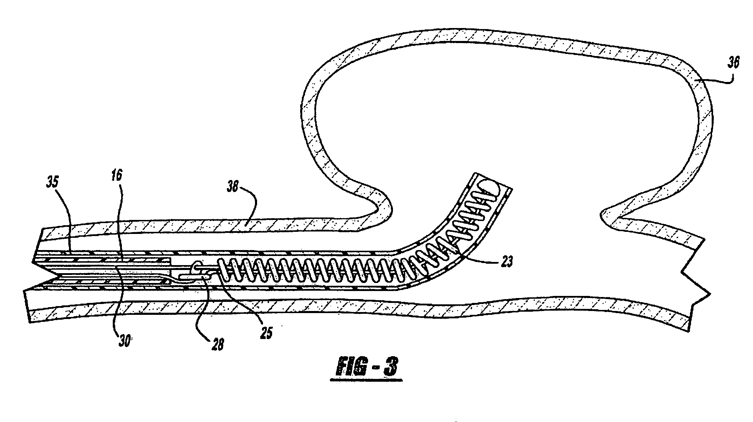

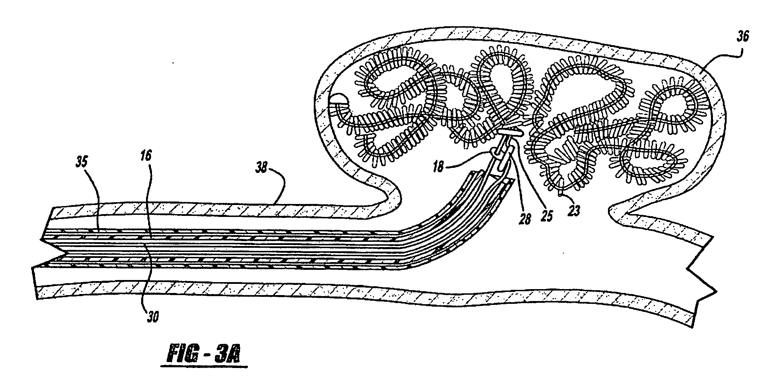

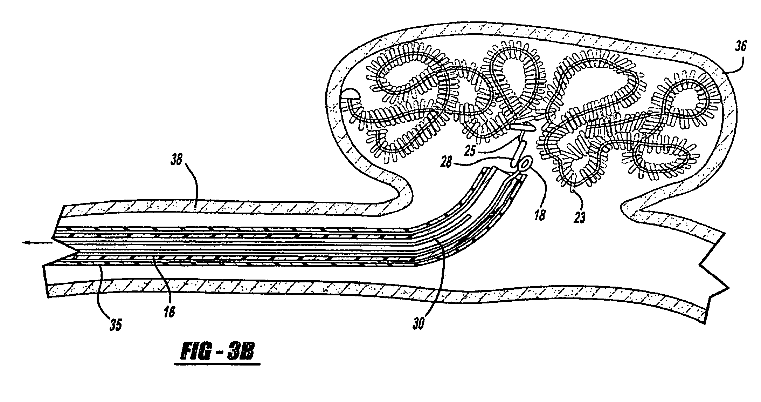

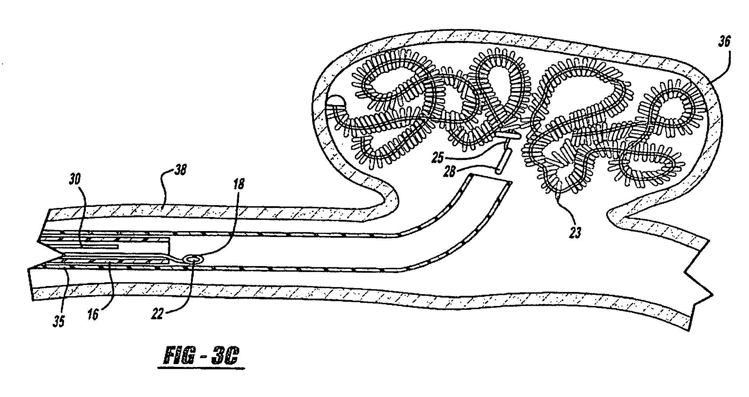

Figures 3, 3A and 3B generally illustrate the sequence of placing an embolic device, such as a helical wound coil into an aneurysm 36 which extends from a vessel wall 38. More particularly, Figure 3 illustrates the vascular occlusive embolic device deployment system 10 in the same configuration as shown in Figure 1 after the pusher member and associated embolic device have been inserted into a delivery catheter 35 and advanced into a position for deployment of the embolic device 23, shown as a helical embolic coil, into the aneurysm 36. Figure 3A illustrates the deployment device having a configuration similar to Figure 2A with the embolic device 23 being placed within the aneurysm 36 but prior to withdrawal of the detachment member 30. At this point, prior to the withdrawal of the detachment member 30, as previously mentioned, if it is determined that the embolic device has been improperly placed, the pusher member may be withdrawn thereby withdrawing the embolic device back into the delivery catheter 35 for repositioning to a different location, or alternatively, to remove the embolic coil entirely from the body.

Figure 3B illustrates the deployment device after the detachment member 30 has been removed from the engagement member 18 thereby releasing the embolic device within the aneurysm 36, and Figure 3C illustrates the deployment device after the pusher member 16 has been withdrawn back into the delivery catheter 35 at the completion of the procedure or alternatively in order to insert a second coil through the delivery catheter 35 and into the same aneurysm.

As is apparent, there are numerous modifications of the preferred embodiment described above which will be readily apparent to one skilled in the art, such as many variations and modifications of the embolic device including numerous coil winding configurations, or alternatively other types of embolic devices. Also, there are many possible variations in the materials and configurations of the release mechanism.

## Claims

1. A vasooclusive embolic device deployment system (10) for use in placing an embolic device (23) at a predetermined site within a vessel (38) comprising:
an elongated flexible deployment catheter (12) having a lumen (14) extending therethrough and having proximal and distal ends;
an elongated pusher member (16) having a lumen extending therethrough and having proximal and distal ends and being slidably disposed within the lumen (14) of the deployment catheter (12);
an embolic device (23) which takes the form of an embolic coil being coupled to a retaining ring (28);
an elongated engagement member (18) being slidably disposed within the lumen of the pusher member and having an aperture (22) extending through the distal end thereof, said engagement member (18) extending through said retaining ring (28); and,
an elongated detachment member (30) extending from a position proximal of the proximal end of the pusher member (16), through the lumen of the pusher member (16) and through the aperture (22) of the engagement member (18) such that when the detachment member (30) is pulled proximally the distal end of the detachment member (30) is withdrawn from the aperture (22) of the engagement member (18) to thereby release the embolic device (23);
**characterised in that** said aperture (22) extends through the distal end of the engagement member (18) such that when said engagement member (18) extends through said retaining ring (28) and when the detachment member (30) extends through said aperture (22), said retaining ring (28) is interlocked onto said engagement member (18) until the detachment member (30) is withdrawn from said aperture (22) releasing said engagement member (18) from said retaining ring.

2. A vasooclusive embolic device deployment system (10) as defined in Claim 1, wherein said embolic coil (23) includes a central lumen extending between proximal and distal sections of the coil (23), the system (10) further comprising a stretch resistant member (25) having first and second ends, said first end of the stretch resistant member (25) is attached to the distal section of the coil (23) and the second end of the stretch resistant member (25) is attached to said retaining ring (28).

3. A vasooclusive embolic device deployment system (10) as defined in Claim 2, wherein said stretch resistant member (25) is formed of a wire,

4. A vasooclusive embolic device deployment system (10) as defined in Claim 2, wherein said stretch resistant member (25) is formed of a fiber.

5. A vasooclusive embolic device deployment system (10) as defined in Claim 2, wherein the distal section of said engagement member (18) is of an L-shaped configuration.

6. A vasooclusive embolic device deployment system (10) as defined in Claim 5, wherein said stretch resistant member (25) is formed of a wire.

7. A vasooclusive embolic device deployment system (10) as defined in Claim 5, wherein said stretch resistant member (25) is formed of a fiber.

## Patentansprüche

1. Einführsystem (10) für eine vasookklusive Embolievorrichtung zum Gebrauch beim Plazieren einer Embolievorrichtung (23) an einem vorbestimmten Ort innerhalb eines Gefäßes (38), das Folgendes umfasst:
einen länglichen flexiblen Einführkatheter (12) mit einem Lumen (14), das sich durch diesen erstreckt, und mit einem proximalen und einem distalen Ende;
ein längliches Drückelement (16) mit einem Lumen, das sich durch dieses erstreckt, und mit einem proximalen und einem distalen Ende, wobei das Druckelement gleitfähig in dem Lumen (14) des Einführkatheters (12) angeordnet ist;
eine Embolievorrichtung (23), welche die Form einer Emboliespirale annimmt, die mit einem Haltering (28) gekoppelt ist;
ein längliches Eingriffselement (18), das gleitfähig in dem Lumen des Drückelements angeordnet ist und eine Öffnung (22) aufweist, die sich durch das distale Ende von diesem erstreckt, wobei das Eingriffselement (18) sich durch den Haltering (28) erstreckt; und
ein längliches Freisetzungselement (30), das sich von einer Position proximal des proximalen Endes des Drückelements (16) durch das Lumen des Drückelements (16) und
durch die Öffnung (22) des Eingriffselements (18) erstreckt, so dass, wenn an dem Freisetzungselement (30) proximal gezogen wird, das distale Ende des Freisetzungselements (30) aus der Öffnung (22) des Eingriffselements (18) zurückgezogen wird, um dabei die Embolievorrichtung (23) zu lösen;
**dadurch gekennzeichnet, dass** sich die Öffnung (22) durch das distale Ende des Eingriffselements (18) erstreckt, so dass, wenn das Eingriffselement (18) sich durch den Haltering (28) erstreckt und wenn das Freisetzungselement (30) sich durch die Öffnung (22) erstreckt, der Haltering (28) auf dem Eingriffselement (18) verriegelt ist bis das Freisetzungselement (30) aus der Öffnung (22) zurückgezogen wird, wobei sich das Eingriffselement (18) von dem Haltering löst.

2. Einführsystem (10) für eine vasookklusive Embolievorrichtung nach Anspruch 1, wobei die Emboliespirale (23) ein zentrales Lumen aufweist, das sich zwischen einem proximalen und einem distalen Abschnitt der Spirale (23) erstreckt, das System (10) weiterhin ein dehnungswiderstandsfähiges Element (25) mit einem ersten und einem zweiten Ende umfasst, wobei das erste Ende des dehnungswiderstandsfähigen Elements (25) an dem distalen Abschnitt der Spirale (23) angebracht ist und das zweite Ende des dehnungswiderstandsfähigen Elements (25) an dem Haltering (28) angebracht ist.

3. Einführsystem (10) für eine vasookklusive Embolievorrichtung nach Anspruch 2, bei welchem das dehnungswiderstandsfähige Element (25) aus einem Draht gebildet ist.

4. Einführsystem (10) für eine vasookklusive Embolievorrichtung nach Anspruch 2, bei welchem das dehnungswiderstandsfähige Element (25) aus einer Faser gebildet ist.

5. Einführsystem (10) für eine vasookklusive Embolievorrichtung nach Anspruch 2, bei welchem der distale Abschnitt des Eingriffelements (18) eine L-förmige Konfiguration aufweist.

6. Einführsystem (10) für eine vasookklusive Embolievorrichtung nach Anspruch 5, bei welchem das dehnungswiderstandsfähige Element (25) aus einem Draht gebildet ist.

7. Einführsystem (10) für eine vasookklusive Embolievorrichtung nach Anspruch 5, bei welchem das dehnungswiderstandsfähige Element (25) aus einer Faser gebildet ist.

## Revendications

1. Système de déploiement d'un dispositif embolique d'occlusion vasculaire (10) à utiliser dans la mise en place d'un dispositif embolique (23) au niveau d'un site prédéterminé dans un vaisseau (38) comprenant :
➢ un cathéter de déploiement flexible allongé (12) ayant un lumen (14) s'étendant dans celui-ci et ayant des extrémités proximale et distale ;
➢ un élément poussoir allongé (16) ayant un lumen s'étendant dans celui-ci et ayant des extrémités proximale et distale et étant disposé de façon à coulisser dans le lumen (14) du cathéter de déploiement (12) ;
➢ un dispositif embolique (23) qui prend la forme d'une spirale embolique qui est couplée à une bague de retenue (28) ;
➢ un élément d'engagement allongé (18) qui est placé de façon à coulisser dans le lumen de l'élément poussoir et ayant une ouverture (22) s'étendant dans l'extrémité distale de celui-ci, ledit élément d'engagement (18) s'étendant dans ladite bague de retenue (28) ; et
➢ un élément de détachement allongé (30) s'étendant depuis une position proximale de l'extrémité proximale de l'élément poussoir (16) par le lumen de l'élément poussoir (16) et par l'ouverture (22) de l'élément d'engagement (18) de sorte que lorsque l'élément de détachement (30) est tiré au niveau proximal, l'extrémité distale de l'élément de détachement (30) est retirée de l'ouverture (22) de l'élément d'engagement (18) pour libérer ainsi le dispositif embolique (23) ;
**caractérisé en ce que** ladite ouverture (22) s'étend dans l'extrémité distale de l'élément d'engagement (18) de sorte que lorsque l'élément d'engagement (18) s'étend dans ladite bague de retenue (28) et que l'élément de détachement (30) s'étend dans ladite ouverture (22), ladite bague de retenue (28) est imbriquée sur ledit élément d'engagement (18) jusqu'à ce que l'élément de détachement (30) soit retiré de ladite ouverture (22) libérant ledit élément d'engagement (18) de ladite bague de retenue.

2. Système de déploiement de dispositif embolique d'occlusion vasculaire (10) tel que défini dans la revendication 1, dans lequel ladite bobine embolique (23) comprend un lumen central s'étendant entre les sections proximale et distale de la bobine (23), le système (10) comprenant en outre un élément résistant à l'étirement (25) ayant une première et une seconde extrémité, ladite première extrémité de l'élément résistant à l'étirement (25) étant liée à la section distale de la bobine (23) et la seconde extrémité de l'élément résistant à l'étirement (25) étant liée à ladite bague de retenue (28).

3. Système de déploiement de dispositif embolique d'occlusion vasculaire (10) tel que défini dans la revendication 2, ledit élément résistant à l'étirement (25) étant formé d'un fil.

4. Système de déploiement de dispositif embolique d'occlusion vasculaire (10) tel que défini dans la revendication 2, ledit élément résistant à l'étirement (25) étant formé d'une fibre.

5. Système de déploiement de dispositif embolique d'occlusion vasculaire (10) tel que défini dans la revendication 2, ladite section distale dudit élément d'engagement (18) présentant une configuration en L.

6. Système de déploiement de dispositif embolique d'occlusion vasculaire (10) tel que défini dans la revendication 5, ledit élément résistant à l'étirement (25) étant formé d'un fil.

7. Système de déploiement de dispositif embolique d'occlusion vasculaire (10) tel que défini dans la revendication 5, ledit élément résistant à l'étirement (25) étant formé d'une fibre.
